# EUROPÄISCHE PATENTSCHRIFT

(11) **EP 1 513 539 B1**
(45) Veröffentlichungstag und Bekanntmachung des Hinweises auf die Patenterteilung: **13.02.2008**
(21) Anmeldenummer: 03760597.9
(22) Anmeldetag: 07.06.2003
(51) Int. Cl.: A61K 31/7072, A61K 31/047, A61P 31/12

(54) **VERWENDUNG VON AMPHIPHILEN NUCLEOSID-PHOSPHONOAMEISENSÄURE-DERIVATEN ZUR BEHANDLUNG VON VIRALEN INFEKTIONSKRANKHEITEN**
USE OF AMPHIPHILIC NUCLEOSIDE PHOSPHONOFORMIC ACID DERIVATIVES FOR THE TREATMENT OF VIRAL INFECTIOUS DISEASES
UTILISATION DE DERIVES AMPHIPHILES D'ACIDE NUCLEOSIDE-PHOSPHONOFORMIQUE POUR TRAITER DES MALADIES INFECTIEUSES VIRALES

(30) Priorität: 19.06.2002 DE 10228059
(43) Veröffentlichungstag der Anmeldung: 16.03.2005
(73) Patentinhaber: Eberhard-Karls-Universität Tübingen Universitätsklinikum, 72076 Tübingen (DE)
(72) Erfinder: HAMPRECHT, Klaus, 72074 Tübingen (DE); SCHOTT, Herbert, 72076 Tübingen (DE); JAHN, Gerhard, 72108 Rottenburg (DE)
(74) Vertreter: Otten, Hajo
(86) Internationale Anmeldenummer: PCT/EP2003/006012
(87) Internationale Veröffentlichungsnummer: WO 2004/000330

(56) Entgegenhaltungen:
- WO-A-00/34298
- DE-A- 4 224 878

## Beschreibung

Die Erfindung betrifft die Verwendung von amphiphilen Nucleosid-Phosphonoameisensäure-Derivaten zur Herstellung eines Arzneimittels zur Behandlung von viralen Infektionskrankheiten ausgelöst durch Adenovirus, humane Cytomegalovirus and Epstein-Barr-Virus.

Virale Infektionen und damit verbundene Erkrankungen stellen insbesondere bei immunsupprimierten Patienten ernstzunehmende Komplikationen dar. Eine Immunsuppression ist beispielsweise bei Transplantationen solider Organe und bei Knochenmark- oder Stammzelltransplantationen zur Vermeidung von Abstoßungsreaktionen vonnöten. Virale Infektionskrankheiten stellen ferner insbesondere für Patienten mit Immunerkrankungen, wie beispielsweise AIDS, ein ernstzunehmendes Risiko dar.

Die Entwicklung antiviraler Pharmaka hat sich als äußerst schwierig erwiesen. Dies beruht v.a. darauf, daß sich Viren nur in Wirtszellen vermehren und dabei als intrazelluläre Parasiten neben viralen Enzymen auch die des Wirtes benutzen. Wegen des komplexen Zusammenwirkens von Virusgenom und Stoffwechsel der Wirtszelle ist es kaum möglich, die Virusreplikation zu stoppen, ohne die Wirtszelle zu schädigen. Darüber hinaus werden viele Virusinfektionen erst dann klinisch manifest, wenn infolge des Virusbefalls die Wirtszellen bereits irreversibel geschädigt sind.

Neben dem humanen Immundefizienz-Virus (HIV) spielen insbesondere einige Mitglieder der Familie Herpesviridae bei Infektionserkrankungen, u.a. von immunsupprimierten Patienten, eine herausragende Rolle. Zu dieser Gruppe zählen bspw. das Herpessimplex-Virus, das Varicella-zoster-Virus (VZV), das Epstein-Barr-Virus (EBV) und das (humane) Cytomegalovirus (HCMV).

Das humane Cytomegalovirus (HCMV) persistiert nach einer Infektion wie auch die anderen Herpes-Viren lebenslang im Wirt. Bei Immunkompetenten verursacht das humane Cytomegalovirus in der Regel ein ähnliches Infektionsbild wie das Pfeiffersche Drüsenfieber. Heiniger et al., berichten, daß es aber beispielsweise bei nicht immunsupprimierten Patienten der chirurgischen Intensivmedizin zu schweren symptomatischen HCMV-Infektion im Sinne einer Organerkrankung kommen kann. Nur ein voll funktionsfähiges Immunsystem kann eine HCMV-Erkrankung im Gefolge einer HCMV-Infektion verhindern.

Zur Chemotherapie bei Virusinfektionen können Inhibitoren der Protein- und der Nukleinsäuresynthese eingesetzt werden. Ein Hauptproblem ist dabei die Toxizität durch Beeinflussung auch des zellulären Mechanismus.

Derzeit stehen nur drei antivirale Wirksubstanzen mit Zulassung zur Therapie einer HCMV-Infektion am Menschen zur Verfügung: Ganciclovir (GCV), Foscarnet (Natrium-Phosphonoameisensäure, PFA) und außerdem Cidofovir (CDV). Ganciclovir ist ein acyclisches Guanosinanalog, Cidofovir ein acyclisches Desoxycytidinmonophosphat (dCMP) -Analog. Foscarnet unterscheidet sich von diesen Nucleosid-/Nucleotidanaloga als Salz der Phosphonoameisensäure strukturell deutlich.

Alle drei Wirkstoffe unterbinden die virale DNA-Synthese letztlich durch Inhibition der HCMV-DNA-Polymerase. 9-[1,3-Di-hydroxy-2-propoxymethyl]guanin (Ganciclovir) muß als Prodrug, um seine antivirale Aktivität zu entfalten, zuerst zum Ganciclovir-Triphosphat phosphoryliert werden. Dazu wird es in einem ersten Schritt durch eine HCMV-kodierte Phosphotransferase (UL97) zu Ganciclovir-Monophosphat phosphoryliert. Anschließend phosphorylieren Kinasen der Wirtszelle dieses Monophosphat über das Diphosphat zum Triphosphat. Das Triphosphat von Ganciclovir wird bei der viralen DNA-Synthese als dGTP-Analog in den nascierenden Strang eingebaut, was zum Kettenabbruch der HCMV-DNA-Replikation führt.

1-[(S)-3-Hydroxy-2-(phosphonomethoxy)propyl]cytosin-Dihydrat (Cidofovir) ist ein acyclisches Nucleosidmonophosphonat, das durch zelluläre Kinasen zum Triphosphat aufphosphoryliert werden muß, um in eine aktive Form überführt zu werden. Die Phosphorylierung wird von Wirtsenzymen durchgeführt und ist somit unabhängig von viruskodierten Kinasen. Der Einbau von CDV-Triphosphat (CDV-TP) in den neu entstehenden viralen DNA-Strang führt wie bei GCV-Triphosphat zum Kettenabbruch der viralen DNA-Replikation.

Foscarnet (Natrium-Phosphonoameisensäure) ist ein Pyrophosphatanalog, das keiner initialen intrazellulären Aktivierung bedarf. Dieser Wirkstoff blockiert direkt die Pyrophosphatbindungsstelle der viralen DNA-Polymerase und inhibiert somit die Abspaltung von Pyrophosphat aus dNTPs.

Die genannten Substanzen verfügen über zum Teil erhebliche Nebenwirkungen. In der Veröffentlichung von Crumpacker CS., "Ganciclovir", N. Engl. J. Med. 1996, 335:721-731, sind die Nebenwirkungen dieser Medikamente zusammengefaßt.

So verursacht die Gabe von Ganciclovir insbesondere eine Verminderung der Anzahl der weißen Blutkörperchen und häufig auch der Thrombocyten. Bei einer Verabreichung über längere Zeit kann auch eine Anämie auftreten. Die Verabreichung von Ganciclovir in voller Dosis in Verbindung mit anderen myelotoxischen Pharmaka kann wegen der hämatologischen Toxizität lebensbedrohlich sein.

Cidofovir besitzt eine dosisabhängige Nephrotoxizität. Sie wird durch ein Ungleichgewicht zwischen schneller Aufnahme in die proximalen Tubuluszellen und dem langsameren Efflux in den Urin hervorgerufen, was zur Akkumulation in den Nieren führt.

Foscarnet besitzt eine erhebliche renale und metabolische Toxizität. Die Nephrotoxizität basiert auf der direkten Schädigung der Nierentubuli.

Eine besonders kritische Situation hinsichtlich erfolgreicher therapeutischer Intervention ergibt sich bei der Manifestation einer Multiresistenz gegen Ganciclovir, Foscarnet und Cidofovir bei einer HCMV-Infektion. Die Resistenzentwicklung ist ein ernstzunehmendes klinisches Problem und kann, insbesondere bei einer Multiresistenz, zum Tode führen. In der Prä-HAART-Ära (Hihly active antiretroviral therapy) waren von der Entwicklung GCV-resistenter Stämme im wesentlichen AIDS-Patienten mit HCMV-Retinitis betroffen.

Beim Nachweis einer Ganciclovir-resistenten HCMV-Infektion wird derzeit als Alternativmedikation Foscarnet eingesetzt. Nachteilig an dieser Vorgehensweise ist jedoch, daß eine relativ hohe Wirkungsdosis an Foscarnet nötig ist, um eine effiziente antivirale Wirkung zu erzielen. Eine hohe Wirkungsdosis wiederum zieht toxische Nebenwirkungen nach sich. Beim Auftreten einer multiresistenten HCMV-Infektion stehen keine weiteren antiviralen Agenzien mehr zur Verfügung.

Weiterhin nachteilig an den derzeit verfügbaren Virostatika ist deren mangelnde orale Bioverfügbarkeit, was eine intravenöse Applikation nötig macht. Die dazu bei einer Dauertherapie eingesetzten Venenkatheter können zu einer Sepsis führen.

Problematisch ist weiterhin die beschränkte Diffusion der hochpolaren Virostatika durch die Blut-Hirnschranke in den Liquorraum. Eckle et al. "Drug-resistant human cytomegalovirus infection in children after allogeneic stem cell transplantation may have different clinical outcomes", Blood 2000, 96(9): 3286-3289, zeigten, daß eine HCMV-Enzephalitis, ausgelöst durch einen multiresistenten HCMV-Stamm, nach einer Knochenmarktransplantation weder durch Ganciclovir intravenös oder intrathekal noch durch Foscarnet oder Cidofovir beeinflußbar war.

Aus der WO98/38202 sind lipophile Phosphonosäuren/Nucleosid-Konjugate bekannt, die eine antivirale Aktivität aufweisen. Die in dieser Druckschrift offenbarten Verbindungen schließen Phosphonoameisensäure/Nucleosid-Konjugate ein, wobei die Konjugate zumindest eine lipophile Gruppe und zumindest eine Nucleosidgruppe enthalten.

Der vorliegenden Erfindung liegt die Aufgabe zugrunde, eine Substanz zur Herstellung eines Arzneimittels bereitzustellen, mit welchem Infektionskrankheiten behandelt werden können, die durch humanpathogene Viren ausgelöst werden, und welches auch bei resistenten und multi-resistenten Virus-Stämmen eine hohe Wirksamkeit aufweist und in geringen Dosen eingesetzt werden kann.

Erfindungsgemäß wird die Aufgabe gelöst durch die Verwendung eines Nucleosid-glycerol-(hydroxycarbonyl)-phosphonats, als Wirkstoff zur Herstellung eines Arzneimittels zur Behandlung von viralen Infektionskrankheiten, die durch humanpathogene Viren ausgelöst werden, die ausgewählt sind aus Adenoviren, humanen Cytomegaloviren, Epstein-Barr-Virus.

Derartige Nucleosid-Phosphonoameisensäure-Derivate sind in der WO 00/34298 beschrieben.

Aus dieser Druckschrift ist es bekannt, Glycerylnucleotide herzustellen, die Kombinationspräparate darstellen, und die bei der Metabolisierung jeweils zwei Wirkstoffe gleichzeitig freisetzten können. Hierbei werden entweder zwei therapeutisch wirksame Nucleosidderivate miteinander oder ein Nucleosidderivat mit der Phosphonoameisensäure oder ihrer Salzform (Foscarnet) über ein Glycerinlipidgerüst kovalent verbunden.

Es ist bevorzugt, wenn als Wirkstoff ein Nucleosid-(5'→2)-1-O-alkyl-rac-glycerol-3-(hydroxycarbonyl)-phosphonat eingesetzt wird, wobei Alkyl ein geradkettiger oder verzweigter gesättigter Rest mit mehr als 6 Kohlenstoffatomen ist.

Einige in der WO 00/34298 beschriebenen Glycerylnucleotide werden zur Behandlung von Krebs und Infektionskrankheiten vorgeschlagen. Die Wirkung eines Nucleosid-(5'→2)-1-O-alkyl-rac-glycerol-3-(hydroxycarbonyl)-phosphonats bei Infektionskrankheiten, die durch humanpathogene Viren hervorgerufen werden, ist jedoch nicht beschrieben.

Die erfindungsgemäß einzusetzenden Nucleosidgruppen sind von Nucleosiden abgeleitet und sind insbesondere nicht natürlich vorkommende Nucleosidgruppen, die einen heterocyclischen Rest aufweisen, der glycosidisch mit einem Zuckerrest verbunden ist.

Als bevorzugter Wirkstoff wird insbesondere 3'-Azido-2',3'-didesoxy-thymidylyl-(5'→2)-1-O-octadecyl-*rac*-glycerol-3-(hydroxycarbonyl)-phosphonat verwendet. Dieser Wirkstoff ist bereits in der WO 00/34298 beschrieben, jedoch ist weder seine Wirkung beschrieben, noch wird sein Einsatz speziell vorgeschlagen.

Die Erfinder konnten in eigenen Versuchen zeigen, daß 3'-Azido-2',3'-didesoxythymidylyl-(5'→2)-1-O-octadecyl-*rac*-glycerol-3-(hydroxycarbonyl)-phosphonat (im folgenden auch mit "N3" bezeichnet) eine antivirale Aktivität aufweist. Die folgende Formel I zeigt die Struktur von N3:

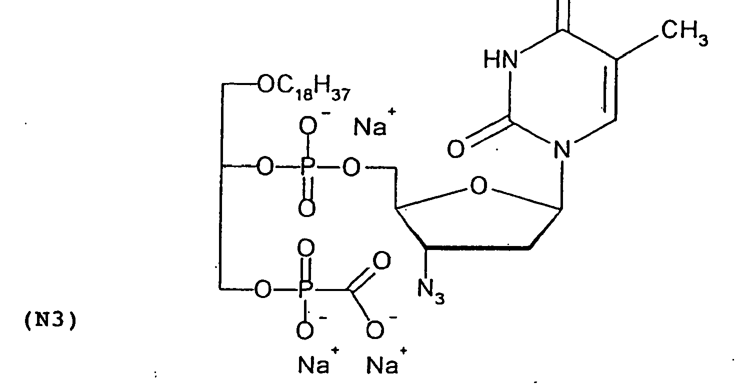

Der Wirkstoff kann zur Herstellung eines Arzneimittels zur Behandlung von Infektionskrankheiten eingesetzt werden, die durch Retroviren, insbesondere durch das HI-Virus, hervorgerufen werden.

HIV-infizierte Menschen tragen wegen der durch das Virus hervorgerufenen Immunschwäche das besondere Risiko von schwerwiegenden Koinfektionen mit Viren, die das Immunsystem gesunder Menschen normalerweise kontrollieren kann. Durch die erfindungsgemäße Verwendung der Wirkstoffe können durch deren Wirkung auf das HI-Virus Folgeinfektionen mit anderen Viren vorteilhaft vermieden werden.

Weiterhin können die Wirkstoffe zur Herstellung eines Arzneimittels zur Behandlung von Infektionskrankheiten eingesetzt werden, die durch Hepatitis-B- und/oder Hepatitis-C-Viren hervorgerufen werden.

Bei einer anderen Anwendung werden die Wirkstoffe zur Herstellung eines Arzneimittels zur Behandlung von Infektionskrankheiten eingesetzt, die durch Adenoviren hervorgerufen werden.

Adenoviren verursachen verschiedene Krankheitsbilder, wie bspw. Infektionen der Luftwege, des Auges und des Intestinaltraktes, die insbesondere bei Kindern schwerwiegende Auswirkungen haben können.

Ferner kann der wirkstoff zur Herstellung eines Arzneimittels zur Behandlung von Infektionskrankheiten eingesetzt werden, die durch humane Beta- und/oder Gamma-Herpesviren, insbesondere durch das Epstein-Barr-Virus und/oder das humane Cytomegalovirus (HCMV) hervorgerufen werden.

Menschen mit besonderem Risiko für schwerwiegende HCMV-Erkrankungen sind HIV-infizierte Patienten, bei denen die Funktion des Immunsystems gestört ist. Patienten, die nach einer Transplantation solider Organe eine medikamentöse Immunsuppression benötigen, sind insbesondere in den ersten drei Monaten ebenfalls gefährdet. Ferner haben Empfänger von Knochenmark-und Stammzelltransplantationen ein hohes Risiko, eine HCMV-Erkrankung zu erleiden, bis das mit der Transplantation übertragene Immunsystem seine volle Funktionsfähigkeit aufgenommen hat.

Insbesondere Neuinfektionen mit HCMV oder eine Reaktivierung kann bei Patienten mit einem supprimierten Immunsystem zu schwersten generalisierten Infektionen mit letalem Ausgang führen. Daher ist die Verwendung der Wirkstoffe bspw. bei AIDS-Patienten mit einer HCMV-Infektion vorteilhaft, da somit zwei Erreger gleichzeitig angegriffen werden können.

In eigenen Versuchen konnten die Erfinder außerdem zeigen, daß zum einen ein erfindungsgemäß eingesetzter Wirkstoff gegenüber Ganciclovir-sensitiven HCMV-Stämmen eine mit Ganciclovir vergleichbare antivirale Aktivität aufwies.

Darüber hinaus erwies sich dieser Wirkstoff gegenüber Ganciclovir-resistenten CMV-Stämmen als hoch aktiv.

Dies ist insbesondere vorteilhaft, wenn eine Infektion mit HCMV-Stämmen vorliegt, die gegen GCV resistent sind, da auch hier keine größere Dosis an dem Wirkstoff im Vergleich zu einem Einsatz bei GCV-sensitiven HCMV-Stämmen eingesetzt werden muß.

Im Vergleich zu Foscarnet besaß der Wirkstoff in weiteren Versuchen eine ca. 40 Mal höhere antivirale Aktivität, selbst gegen Ganciclovir-resistente HCMV-Stämme.

Dies ist besonders vorteilhaft, da es bislang zwar möglich war, mit Foscarnet die Replikation Ganciclovir-resistenter HCMV-Stämme *in vitro* und *in vivo* zu beeinflussen, jedoch nicht mit einer so niedrigen molaren Konzentration wie bei Verwendung von N3. Hier ist besonders vorteilhaft, daß dadurch die starken Nebenwirkungen, insbesondere die Nephrotoxizität, die durch andauernde Verabreichung von Foscarnet hervorgerufen werden, reduziert werden können.

Bei der Verwendung von Nucleosid-Phosphonoameisensäure-Derivaten zur Herstellung von Arzneimitteln ist weiterhin vorteilhaft, daß die additive myelotoxische Wirkung von Ganciclovir und Azidothymidin und die dadurch begrenzten Therapiemöglichkeiten überwunden werden können.

In anderen bevorzugten Verwendungen wird der Wirkstoff bei durch multiresistente HCMV-Stämme hervorgerufenen Erkrankungen eingesetzt.

So beschreiben bspw. Eckle et al., Drug-resistant human cytomegalovirus infection in children after allogenic stem cell transplantation may have different clinical outcomes, Blood 2000, 96(9):3286-3289, einen HCMV-Stamm, der eine Multiresistenz gegenüber Ganciclovir, Foscarnet und Cidovir entwickelte.

Da bislang noch kein Wirkstoff gegen multiresistente HCMV-Stämme gefunden werden konnte, der eine geringere Toxizität, jedoch eine gleiche Wirksamkeit wie bspw. Foscarnet aufweist, stellen die Nucleosid-Phosphonoameisensäure-Derivate äußerst wirksame Substanzen für die Bekämpfung von multiresistenten HCMV-Stämmen dar.

Nucleosid-Phosphonoameisensäure-Derivate können bei durch HCMVhervorgerufene Erkrankungen wie Pneumonie, Hepatitis, Colitis, Retinitis und Enzephalitis eingesetzt werden. Diese Krankheiten stellen insbesondere für immungeschwächten Patienten eine große Gefahr dar.

In einer Ausführungsform wird das den Wirkstoff enthaltende Arzneimittel parenteral und/oder oral verabreicht.

Dabei kann der Wirkstoff zusammen mit einem oder mehreren pharmazeutisch akzeptablen Trägern verabreicht werden.

Unter "Trägern" oder Hilfsstoffen werden hierbei solche Substanzen verstanden, die in der Arzneimittelherstellung üblicherweise als Verdünnungsmittel, Bindemittel, Suspensionsmittel, Schmiermittel, Stabilisatoren usw. entweder als Lösung oder als feste Substanz verwendet werden. Dazu zählen, sind aber nicht hierauf beschränkt, bspw. Wasser, Salzlösungen, Alkohole, pflanzliche Öle, Polyethylenglycole, Gelatine, Lactose, Glucose, Amylose, Cellulose, Glycerol, Magnesiumstearat, Albumin, Monoglyceride, Diglyceride, Polyvinylpyrrolidon, etc. Eine Reihe von weiteren geeigneten Substanzen findet sich in A. Kibbe, Handbook of pharmaceutical excipients, 3. Ed., 2000, American Pharmaceutical Association and pharmaceutical press.

Die pharmazeutische Zubereitung kann, wenn erwünscht, sterilisiert werden und kann außerdem Konservierungsmittel, Stabilisierungs- und/oder Dispersionsmitel, Emulgatoren, Puffer, Farbstoffe, Geschmacksstoffe, etc. enthalten, die mit der aktiven Verbindung nicht interferieren.

Orale Verabreichungen können in Form von bspw. Tabletten, Kapseln, Pastillen oder als flüssige Zubereitungen in Form eines Sirups vorliegen, die nach bekannten Verfahren, bspw. unter Zusatz von Kohlenhydrat-Bindemitteln wie Maisstärke etc., hergestellt werden. Für eine parenterale Verabreichung kann die Substanz als Injektion oder Infusion ebenfalls nach bekannten Verfahren formuliert werden.

Der Wirkstoff kann dabei auch bspw. in Liposomen und/oder Nanopartikel eingebaut werden, wobei die jeweils eingeführten lipophilen Reste die Größe und Stabilität der Liposomen maßgeblich beeinflussen.

Der Wirkstoff kann darüber hinaus auch in Verbindung mit anderen Wirkstoffen oder Arzneimitteln bei einer therapeutischen Anwendung eingesetzt werden, wie bspw. Reverse-Transkriptase-Hemmer, Protease-Hemmer, Interferon oder Interleukin II, oder bei immunmodulierenden Therapien wie Knochenmarks- oder Stammzelltransplantationen/Transplantationen solider Organe, oder mit Wirkstoffen, die die Anzahl der Lymphocyten steigern. Es können auch gleichzeitig mehrere Nucleosid-Phosphonoameisensäure-Derivate in einer Formulierung eingesetzt werden.

Eine genaue Dosierung hängt dabei vom Verabreichungsweg, dem zu behandelnden Zustand, der Schwere der Krankheit, dem Gewicht und dem Alter des zu behandelnden Patienten, etc. ab.

Es versteht sich, daß die vorstehend beschriebenen und nachstehend noch zu erläuternden Merkmale nicht nur in der jeweils angegebenen Kombination, sondern auch in anderen Kombinationen oder auch in Alleinstellung verwendbar sind, ohne den Rahmen der vorliegenden Erfindung zu verlassen.

Weitere Vorteile ergeben sich aus den Beispielen.

### Beispiele:

### Darstellung von verestertem und unverestertem 3'-Azido-2',3'-didesoxythymidylyl-(5'→2)-1-O-octadecyl-rac-glycerol-3-(hydroxycarbonyl)-phosphonat

Auf der Basis der in der WO 00/34298 beschriebenen Synthese wurde 3'-Azido-2',3'-didesoxythymidylyl-(5'→2)-1-O-octadecyl-rac-glycerol-3-(hydroxycarbonyl)-phosphonat (im folgenden N3) in drei Schritten hergestellt:

### 1. Schritt:

15,8 g (23 mmol) 3-O-(4-Monomethoxytrityl)-1-O-octadecyl-rac-glycerol-2-hydrogenphosphonat wurden zusammen mit 8 g (30 mmol) 3'-Azido-2',3'-didesoxythymidin in 70 ml wasserfreiem Pyridin gelöst. Anschließend wurde die Lösung auf 10°C abgekühlt und unter Feuchtigkeitsausschluß mit 18 ml (148 mmol) Pivaloylchlorid versetzt und 7 min bei Raumtemperatur gerührt. Das anschließend auf 0°C abgekühlte Gemisch wurde mit 18 ml Wasser und 105 ml einer 0,2 M Lösung von Iod in THF versetzt und ohne Kühlung für 1 h gerührt. Vor der Konzentration des Reaktionsansatzes im Rotationsverdampfer zu einem Sirup wurde das überschüssige Iod durch Zusatz von festem Natriumhydrogensulfit reduziert. Der Sirup wurde in 150 ml Chloroform aufgenommen und dreimal gegen 450 ml einer Wasser/Methanol/NaCl_{gesättigt}-Mischung 1:2:1 (v/v/v) ausgeschüttelt. Die organische Phase wurde wiederum zu einem Sirup konzentriert, der noch dreimal mit je 200 ml Toluol coevaporiert wurde. Um die 4-Monomethoxytritylgruppe gegen Wasserstoff auszutauschen, wurde der Sirup in 150 ml Essigsäure/Wasser 4:1 (v/v) gelöst, 15 min auf 50°C erwärmt und anschließend im Rotationsverdampfer wieder zum Sirup konzentriert. Durch Coevaporation mit Toluol im Ölpumpenvakuum wurde die Essigsäure entfernt, der hierbei erhaltene Sirup anschließend in 100 ml Chloroform/Methanol (95:5) (v/v) gelöst und an einer Kieselgelsäule mit einem Chloroform/Methanol-Gradienten chromatographisch gereinigt. Von den vereinigten produktenthaltenden Fraktionen blieb nach dem Abrotieren des Lösungsmittels ein Feststoff zurück, der aus Methanol bei -25°C umkristallisiert wurde, wodurch 10,5 g (15,6 mmol) 3'-Azido-2',3'-didesoxythymidylyl-(5'→2)-1-O-octadecyl-*rac*-glycerol als Vorstufe des wirkstoffs erhalten wurden. Diese Vorstufe wies auf der Kieselgeldünnschichtplatte im Laufmittelsystem Chloroform/Methanol 7:3 (v/v) einen R_{f}-Wert von 0,5 auf.

### 2. Schritt:

2,3 g (12 mmol) (Ethoxycarbonyl)phosphorigsäuredichlorid wurden in 30 ml Trimethylphosphat gelöst und unter Rühren im Eisbad auf ca. 0°C gekühlt. Anschließend wurden unter Rühren 4,0 g (6 mmol) 3'-Azido-2',3'-didesoxythymidylyl-(5'→2)-1-O-octadecyl-rac-glycerol zugegeben und zuerst weitere 5 h im Eisbad (0°C), dann über Nacht bei Raumtemperatur unter Feuchtigkeitsausschluß gerührt. Die Reaktionsmischung wurde dann in 120 ml Diethylether gelöst und zweimal gegen je 100 ml H₂O ausgeschüttelt. Die saure wässrige Phase wurde im Eisbad mit 2 M Natronlauge auf pH 6,5 eingestellt, konzentriert und einmal mit Toluol coevaporiert. Der Rückstand wurde in 100 ml Diethylether suspendiert und zur Kristallisation bei 4°C verwahrt. Der erhaltene Niederschlag wurde abgesaugt, mit kaltem Diethylether gewaschen und getrocknet. Die Mutterlauge wurde zur Nachfällung auf ca. 1/3 des Ausgangsvolumens konzentriert und der erneut erhaltene Niederschlag analog aufgearbeitet. Die vereinigten Niederschläge (Rohprodukt) wurden in 0,05 M Ammoniumacetat/Methanol (2:8) (v/v) gelöst und an einer Reversed-Phase (RP₁₈-Säule) in einem Ammoniumacetat/Methanol-Gradienten chromatographisch gereinigt. Die vereinigten produktenthaltenden Fraktionen wurden auf ca. 1/4 des Ausgangsvolumens konzentriert und anschließend lyophilisiert. Man erhält 3,2 g 3'-Azido-2',3'-didesoxythymidylyl-(5'→2)-1-O-octadecyl-*rac*-glycerol-3-(hydroxycarbonyl)-phosphonatethylester. Das Produkt ist die veresterte Form von N3 und wird im folgenden als N4 bezeichnet.

### 3. Schritt:

2 g 3'-Azido-2',3'-didesoxythymidylyl-(5'→2)-1-O-octadecyl-*rac*-glycerol-3-(hydroxycarbonyl)-phosphonatethylester (N4) wurden in 50 ml Wasser gelöst und mit 107 ml 1 M Natronlauge versetzt und nach 10 min. Rühren bei Raumtemperatur mit einem Kationenaustauscher auf pH 7 neutralisiert. Die abgesaugte Lösung wurde mit Natronlauge auf pH 7,5 bis 8 eingestellt und anschließend lyophilisiert. Das Lyophilisat wurde an einer Reversed-Phase (RP₁₈-Säule) in einem Ammoniumacetat/Methanol-Gradienten chromatographisch gereinigt und ergab 1,5 g 3'-Azido-2',3'-didesoxythymidylyl-(5'→2)-1-O-octadecyl-*rac-*glycerol-3-(hydroxycarbonyl)-phosphonat (N3) in Form eines farblosen Pulvers, das auf der Kieselqeldünnschichtplatte im Laufmittelsystem Chloroform/Methanol 6:4 (v/v) einen R_{f}-Wert von 0,1 aufwies.

### Nachweis der HCMV-spezifischen antiviralen Aktivität

Um die HCMV-spezifische antivirale Aktivität von N3 nachzuweisen, wurde ein modifizierter zelladaptierter Plaque-Reduktionsassay durchgeführt. Dafür eingesetzt wurden Kokulturen von infizierten und nicht infizierten humanen Vorhautfibroblasten (HFF).

In Prix et al., A simplified assay for screening of drug resistance of cell-associated cytomegalovirus strains, Journal of Clinical Virology 1998, 11: 29-37, und Prix et al., Comprehensive restriction analysis of the UL97 region allows early detection of ganciclovir-resistant human cytomegalovirus in an immunocompromised child, Journal of infectious deseases 1999, 180: 491-495, ist die Durchführung des Assays zu ersten Mal beschrieben und wird nachstehend kurz dargestellt.

### Vorbereitung der HFF-Zellen

HCMV-Primärisolate auf HFF-Zellen in Röhrchenkultur wurden auf kleine Zellkulturflaschen (25 cm²) mit dichtem HFF-Zellrasen umgesetzt und kultiviert. Nach Erreichen eines ca. 20 - 40%igen zytopathischen Effekts (CPE) wurden die Zellen mit Phosphatgepufferter Kochsalzlösung (PBS) gewaschen und der Zellrasen abtrypsiniert. Nach Trypsininaktivierung wurde das Pellet in Eagles minimalem essentiellen Medium (MEM)-10% FCS (Fetales Kälberserum) aufgenommen und zur Trennung von Zellaggregaten schonend filtriert.

Parallel wurden die nicht infizierten HFF-Zellen auf 175 cm²-Schalen kultiviert, gewaschen, abtrypsiniert und nach weiteren Reinigungsschritten in MEM-10% FCS aufgenommen.

### Herstellung der Kokulturen

Zur Herstellung von Kokulturen wurden die infizierten Zellen mit den nicht infizierten folgendermaßen vermischt: 2,5 x 10⁴ nicht infizierte Zellen/100 µl wurden in zwei unterschiedlichen Verhältnissen, mit 500 und 1000 infizierten Zellen /100 µl gemischt. Von jeder dieser Verdünnungsstufen wurden jeweils 100 µl in einer Dreifachbestimmung auf eine 96-well Mitkrotiterplatte aufgetragen. Zur Zelladhärenz an den Mikrotiterplattenboden wurden die Kokulturen mindestens 4 h lang inkubiert, bevor das Medium vorsichtig entfernt wurde.

### Auftragen der Virostatika

Unmittelbar nach Entfernen des Mediums wurden bereits vorher vorbereitete Virostatika-MEM-Lösungen mit steigender Ganciclovir- (GCV), Foscarnet- (PFA) oder 3'-Azido-2',3'-didesoxy-thymidylyl-(5'→2)-1-O-octadecyl-rac-glycerol-3-(hydroxycarbonyl)-phosphonat (im folgenden: N3)-Konzentration in 200 µl-Portionen pro Well auf die Mikrotiterplatte aufgetragen.

Anschließend wurden die Kulturen 4 Tage lang inkubiert, so daß sich die Viren von den infizierten Einzelzellen in Form von Plaquebildung durch Infektion der Nachbarzellen ausbreiten konnten.

### HCMV-Nachweis mittels in situ-ELISA

Im Anschluß an die 4-tägige Inkubation wurde das Medium schonend abgegossen und die Mikrokulturen mit 200 µl eiskaltem (-20°C) Methanol permeabilisiert und fixiert. Nach 20 min wurde das Methanol entfernt und die Zellen zweimal mit PBS gewaschen.

Anschließend wurde der primäre Antikörper (anti-IE1-pp72, E13 Klon, Paesel & Lorei, Deutschland) aufgetragen, der gegen das HCMV Immediate Early Antigen (UL123) von HCMV gerichtet ist. Nach 1 h Inkubation wurden die Zellen gewaschen und mit dem sekundären Antikörper (Meerrettich-Peroxidase konjugierter Maus-HCMV-IgG-Antikörper; DAKO, Dänemark) für 1 h inkubiert. Nach Zugabe des Peroxidase/H₂O₂-Substrats wurde durch die in situ Braunfärbung das Immediate Early Antigen von HCMV in den Kernen infizierter Fibroblasten unter dem Mikroskop nachgewiesen. Das Ergebnis wurde in ausgewählten Beispielen fotographisch dokumentiert.

Bei GCV wurden die Konzentrationen 0 und 5 µM (Meßreihe 0 bis 50 µM), bei PFA 0 und 300 µM (150-500 µM) und bei N3 6,25 µM (3-50 µM) dokumentiert. Ebenso eingesetzt wurden 6,25 µM der strukturidentischen Ethyl-veresterten Verbindung N4.

### Ergebnisse:

### GCV-resistenter und PFA/CDV-sensitiver HCMV-Stamm

Bei diesem Versuch wurden 500 HCMV-infizierte HFF-Zellen/Mikrokultur eingesetzt. Durch 6,25 µM N3 fand im Vergleich zu 5 µM GCV eine starke Plaquereduktion statt. Auch der Vergleich der mittleren Plaquezahlen/Mikrokultur (GCV: 19,5; N3: 5,7) zeigte, daß die Verbindung N3 sowohl die Plaquezahl als auch deren Größe äußerst wirkungsvoll reduzierte (Ergebnisse nicht gezeigt).

Bei einem Vergleich der Wirkung von N3 zu PFA auf die Plaquereduzierung zeigte sich deutlich, daß sowohl PFA (Anzahl der Plaques: 5) als auch N3 (7,5) im Vergleich zur Negativ-Kontrolle mit 0 µM PFA (37,9) die Plaquezahl und -größe deutlich reduzierten, jedoch mußte eine bedeutend höhere Konzentration an PFA eingesetzt werden, um eine mit N3 vergleichbare Wirksamkeit zu erzielen (300 µM PFA im vergleich zu 6,25 µM N3).

### Multiresistenter HCMV-Stamm

Der verwendete HCMV-Stamm war GCV-/PFA-/CDV-resistent (= multiresistenter HCMV-Stamm, siehe Eckle et al., Drug-resistant human cytomegalovirus infection in children after allogenic stem cell transplantation may have different clinical outcomes, Blood 2000, 96(9):3286-3289). Für die Kokultur eingesetzt wurden hier 1500 HCMV-infizierte HFF-Zellen/Mikrokultur.

Durch den Vergleich der mittleren Plaquezahlen wurde deutlich, daß N3 hochaktiv gegen den multiresistenten HCMV-Stamm ist (20,5 für PFA im Vergleich zu 2,3 für N3).

### Auswertung

Für eine korrekte Auswertung der Platten galt, daß ein Plaque aus einem Zellverband von mindestens 10 Einzelzellen bestehen mußte, um in der Zählung berücksichtigt zu werden. Die ID₅₀ Werte, basierend auf der mittleren Plaque-Anzahl, wurden durch nicht lineare Regressionsanalyse mittels der Probit-Analyse (statistical software package, SPSS, München) berechnet, wobei die Konzentration der Verbindung bestimmt wurde, die notwendig ist, um die Anzahl der Plaques um 50 % zu reduzieren (ID₅₀ Werte).

In einem Vorscreening zeigte sich, daß ein Nucleosid-(5'→2)-1-O-octadecyl-rac-glycerol-3-(hydroxycarbonyl)-phosphonat bei Veresterung der Hydroxycarbonylphosphonatgruppe (= N4) in vitro eine deutlich verminderte antivirale Wirkung aufwies.

Die nachfolgende Tabelle 1 zeigt die zusammengefaßten Ergebnisse der Ermittlung der ID₅₀-Werte sowie des 95 % Konfidenzintervalls der jeweils angegebenen Untersuchungsreihen gegenüber einem GCV-sensitiven HCMV-Stamm (Spalte 1) und gegenüber einem GCV-resistenten HCMV-Stamm (Spalte 2) bezüglich der Verwendungen von GCV und N3.

**Tabelle 1: Antivirale Aktivität von N3 vs. GCV**

| **SUBSTANZ** | **LD₅₀ (95%CI): µM** (GCVₛₑₙₛ/PFAₛₑₙₛ/CDVₛₑₙₛ) N_{iz} = 500/Mikrokultur | | **ID₅₀ (95%CI) : µM** (GCVᵣₑₛ/PFAₛₑₙₛ/CDVₛₑₙₛ) N_{iz} = 1000/Mikrokultur | |
|---|---|---|---|---|
| GCV | 2,8 (1,5 - 4,4) | 1,96 (0,2 - 4,1) | 14,3 (12,0 - 76,0) | 13,6 (4,5 - 72,6) |
| N3 | 4,0 (2,3 - 6,2) | | 1,3 (0,5 - 2,2) | 2,2 (0,9 - 3,5) |

Wie aus Spalte 1 der Tabelle ersichtlich ist, weist N3 bei einem GCV-/PFA-/CDV-sensitiven HCMV-Stamm eine mit GCV vergleichbare antivirale Aktivität auf (2,8 µM für GCV im Vergleich zu 4,0 µM). Eingesetzt wurden hier jeweils 500 infizierte Zellen/Mikrokultur.

Gegen den GCV-resistenten HCMV-Stamm ist N3 hochaktiv, was sich in einer ca. 11 x geringeren ID₅₀ im Vergleich zu GCV äußert (14,3 für GCV im Vergleich zu 1,3 für N3), wobei hier 1000 infizierte Zellen/Mikrokultur eingesetzt wurden.

Nachstehend faßt die Tabelle 2 die ermittelten ID₅₀ Werte und das 95 % Konfidenzintervall von N3 im Vergleich mit PFA selbst bezüglich eines GCV-resistenten HCMV-Stammes zusammen.

**Tabelle 2: Antivirale Aktivität von N3 vs. PFA gegen einen GCV-resistenten HCMV-Stamm**

| **SUBSTANZ** | **ID₅₀ (95%CI): µM** (GCVᵣₑₛ/PFAₛₑₙₛ/CDVₛₑₙₛ) N_{iz} = 1000/Mikrokultur | |
|---|---|---|
| PFA | 68,8 (10,4 - 111,4) | 62,9 (43,2 - 81,1) |
| N3 | 1,8 (0,5 - 3,2) | 1,6 (0,5 - 2,9) |

Die in der Tabelle 2 zusammengefaßten Ergebnisse (Mehrfachbestimmungen) zeigen, daß N3 eine ca. 40 x höhere antivirale Aktivität als PFA selbst gegen einen GCV-resistenten/PFAsensitiven HCMV-Stamm besitzt (64,8 für PFA im Vergleich zu 1,8 für N3). Dies bedeutet, daß zur Erreichung einer antiviralen Wirksamkeit für N3 eine bedeutend geringere Menge an Wirkstoff eingesetzt werden mußte als für PFA selbst. Eingesetzt wurden 1000 infizierte HFF-Zellen/Mikrokultur.

Tabelle 3 zeigt nachstehend die Ergebnisse für die ID₅₀ Werte und des 95 % Konfidenzintervalls von N3 im Vergleich mit PFA gegenüber einem multiresistenten HCMV-Stamm, d.h. einem GCV-, PFA- und CDV-resistenten Stamm.

**Tabelle 3: Antivirale Aktivität von N3 vs. PFA gegen einen multiresistenten HCMV-Stamm**

| SUBSTANZ | **ID₅₀ (95%CI): µM** Multiresistent: GCVᵣₑₛ/PFAᵣₑₛ/CDVᵣₑₛ N_{iz} = 1500/Mikrokultur | | |
|---|---|---|---|
| PFA | 309,7 (239,9 - 394,4) | 336,5 (294,8 - 390) | 474,1 (319 - 1098) |
| N3 | 4,0 (1,2 - 6,8) | 6,5 (5,1 - 7,8) | 2,4 (1,0 - 3,2) |
| **ID_{50PFA}/ ID_{50N3}** | 77,4 | 51,8 | 197, 5 |

In der Tabelle 3 sind die Ergebnisse von drei Versuchsreihen dargestellt. Sie zeigen, daß N3 gegen einen multiresistenten HCMV-Stamm hochaktiv ist, was sich in einem deutlich geringeren ID₅₀ Wert für N3 im Vergleich zu PFA und dadurch in einem hohen ID_{50pFA}/ ID_{50N3} Verhältnis äußert (ID_{50PFA}/ ID_{50N3}: 50 - 200). Eingesetzt wurden 1500 infizierte Zellen/Mikrokultur.

Zusammengefaßt konnten die Erfinder somit zeigen, daß N3 bei der Behandlung viraler Infektionskrankheiten aufgrund der hohen Wirksamkeit eine äußerst wirksame Alternative im Vergleich zu Ganciclovir, Foscarnet und Cidovir darstellt. Eine an der Hydroxycarbonylphosphonatgruppe mit Ethyl veresterte Verbindung (= N4), die ansonsten mit N3 strukturidentisch ist, wies keine antivirale Aktivität gegen multiresistente Stämme auf. Darüber hinaus ist N3 bei teilweise resistenten Viren aufgrund der hohen antiviralen Aktivität in bereits geringen Dosen der Substanz PFA vorzuziehen, da durch die Verwendung von N3 die hohe Nephrotoxizität von PFA insbesondere bei fortgesetzt hohen Dosen vermieden werden kann.

Weiterhin konnten die Erfinder zeigen, daß die Substanz N3 bei multiresistenten HCMV-Stämmen eine antivirale Aktivität aufwies und die Verwendung der Substanz somit eine einzigartige Behandlungsmöglichkeit bei Erkrankungen bietet, die durch multiresistente Viren-Stämme hervorgerufen werden.

Da die cytotoxische Wirkung von N3 *in vitro* gegenüber humanen Vorhautfibroblasten bei Konzentrationen von >125 µM bestimmt werden konnte, ist somit auch eine ausreichende therapeutische Breite für einen *in vivo* Einsatz gegeben.

## Patentansprüche

1. Verwendung eines Nucleosid-(5'→2)-1-O-alkyl-*rac*-glycerol-3-(hydroxycarbonyl)-phosphonats als Wirkstoff zur Herstellung eines Arzneimittels zur Behandlung von Infektionskrankheiten, die durch humanpathogene Viren hervorgerufen werden, **dadurch gekennzeichnet, daß** das Arzneimittel zur Behandlung von Infektionskrankheiten eingesetzt wird, die durch Viren hervorgerufen werden, die ausgewählt sind aus Adenovirus, humanes Cytomegalovirus und Epstein-Barr-Virus.

2. Verwendung nach Anspruch 1, **dadurch gekennzeichnet, daß** als Wirkstoff 3'-Azido-2',3'-didesoxythymidylyl-(5'→2)-1-O-octadecyl-rac-glycerol-3-(hydroxycarbonyl)-phosphonat eingesetzt wird.

3. Verwendung nach Anspruch 1 oder 2, **dadurch gekennzeichnet, daß** die Substanz zur Herstellung eines Arzneimittels zur Behandlung von Infektionskrankheiten eingesetzt wird, die durch Ganciclovir-sensitive humane Cytomegalovirus-Stämme hervorgerufen werden.

4. Verwendung nach Anspruch 1 oder 2, **dadurch gekennzeichnet, daß** die Substanz zur Herstellung eines Arzneimittels zur Behandlung von Infektionskrankheiten eingesetzt wird, die durch Ganciclovir-resistente humane Cytomegalovirus-Stämme hervorgerufen werden.

5. Verwendung nach Anspruch 1 oder 2, **dadurch gekennzeichnet, daß** die Substanz zur Herstellung eines Arzneimittels zur Behandlung von Infektionskrankheiten eingesetzt wird, die durch multiresistente HCMV-Stämme hervorgerufen werden.

6. Verwendung nach einem der Ansprüche 1 bis 5, **dadurch gekennzeichnet, daß** das Arzneimittel so hergestellt ist, daß es parenteral und/oder oral verabreicht werden kann.

## Claims

1. Use of a nucleoside-(5'→2)-1-O-alkyl-*rac*-glycerol-3-(hydroxycarbonyl)-phosphonate, as active compound for the manufacture of a medicament for treating infectious diseases which are elicited by viruses which are pathogenic to humans, **characterized in that** the medicament is used for treating infectious diseases which are elicited by viruses which are selected from adenovirus, human cytomegalovirus and Epstein-Barr virus.

2. Use according to claim 1, **characterized in that** use is made, as active compound, of 3'-azido-2',3'-dideoxythymidylyl-(5'→2)-1-O-octadecyl-*rac*-glycerol-3-(hydroxycarbonyl)-phosphonate.

3. Use according to claim 1 or 2, **characterized in that** the substance is used for the manufacture of a medicament for treating infectious diseases which are elicited by ganciclovir-sensitive human cytomegalovirus strains.

4. Use according to claim 1 or 2, **characterized in that** the substance is used for the manufacture of a medicament for treating infectious diseases which are elicited by ganciclovir-resistant human cytomegalovirus strains.

5. Use according to claim 1 or 2, **characterized in that** the substance is used for the manufacture of a medicament for treating infectious diseases which are elicited by multiresistant HCMV strains.

6. Use according to one of claims 1 to 5, **characterized in that** the medicament is produced such that it can be administered parenterally and/or orally.

## Revendications

1. Utilisation d'un nucléoside-(5'→2)-1-O-alkyl-*rac*-glycérol-3-(hydroxycarbonyl)-phosphonate comme principe actif pour la production d'un médicament pour le traitement des maladies infectieuses qui sont provoquées par des virus pathogènes pour l'homme, **caractérisée en ce que** le médicament est utilisé pour le traitement des maladies infectieuses qui sont provoquées par des virus qui sont choisis parmi les adénovirus, le cytomégalovirus humain et le virus d'Epstein-Barr.

2. Utilisation selon la revendication 1 **caractérisée en ce que** le 3'-azido-2',3'-didésoxythymidylyl-(5'→2)-1-O-octadécyl-*rac*-glycérol-3-(hydroxy-carbonyl)-phosphonate est utilisé comme principe actif.

3. Utilisation selon la revendication 1 ou 2 **caractérisée en ce que** la substance est utilisée pour la production d'un médicament pour le traitement des maladies infectieuses qui sont provoquées par des souches de cytomégalovirus humain sensibles au ganciclovir.

4. Utilisation selon la revendication 1 ou 2 **caractérisée en ce que** la substance est utilisée pour la production d'un médicament pour le traitement des maladies infectieuses qui sont provoquées par des souches de cytomégalovirus humain résistantes au ganciclovir.

5. Utilisation selon la revendication 1 ou 2 **caractérisée en ce que** la substance est utilisée pour la production d'un médicament pour le traitement des maladies infectieuses qui sont provoquées par des souches de HCMV multirésistantes.

6. Utilisation selon l'une des revendications 1 à 5 **caractérisée en ce que** le médicament est produit de telle manière qu'il peut être administré par voie parentérale et/ou orale.
